# EUROPEAN PATENT APPLICATION

(11) **EP 4 806 492 A1**
(43) Date of publication of application: **16.09.2026**
(21) Application number: 23958320.6
(22) Date of filing: 10.11.2023
(51) Int. Cl.: A61M 25/09

(54) **MEDICAL DEVICE**

(71) Applicant: ASAHI INTECC CO., LTD., Seto-shi, Aichi, 489-0071 (JP)
(72) Inventor: FUKUSHIMA, Kensei, Seto-shi, Aichi 489-0071 (JP)
(74) Representative: Prinz & Partner mbB
(86) International application number: PCT/JP2023/040504
(87) International publication number: WO 2025/099919

(57) **Abstract**

A medical device includes a core shaft. The medical device satisfies at least one of the following requirement (A) and requirement (B). Requirement (A): y ≥ 0.0056x (where x is a distal-end load (mN), and y is a measured torque value (mN·m)). Requirement (B): x ≤ 4.5 and y ≥ 0.020.

## Description

### TECHNICAL FIELD

The technology disclosed in the present specification relates to a medical device.

### BACKGROUND ART

Methods using catheters are widely performed to treat or examine, for example, a stenosed portion or an occluded portion (hereinafter referred to as a "lesion portion") in a blood vessel. A guidewire is used to guide the catheter to the lesion portion in the blood vessel.

Patent Literature 1 discloses a procedure of guiding a guidewire to a branched blood vessel through a strut of a stent.

### CITATION LIST

### Patent Literature

Patent Literature 1: U.S. Patent Application Publication No. 2004/0225345

### DISCLOSURE OF INVENTION

### TECHNICAL PROBLEM

In a procedure using a guidewire, when a proximal end portion of the guidewire is rotated, a distal end portion of the guidewire may not rotate linearly, and a phenomenon called "whip" may occur in which the distal end portion rotates all at once when the proximal end portion is further rotated. When whip occurs, for example, a procedure of selecting a branched blood vessel using the guidewire becomes difficult. Conventional guidewires suppress whip by the cross-sectional shape of the core shaft. However, the inventor recognized a problem that whip cannot be sufficiently suppressed depending on the cross-sectional shape of the core shaft. Such a problem is not limited to guidewires but is a common problem in medical devices.

The technology that can solve the above-described problem is disclosed in the present specification.

### SOLUTION TO PROBLEM

(1) A medical device (100) disclosed in the present specification includes a core shaft (10) and satisfies at least one of the following requirement (A) and requirement (B). Requirement (A): y ≥ 0.0056x (where x is a distal-end load (mN), and y is a measured torque value (mN·m)). Requirement (B): x ≤ 4.5 and y ≥ 0.020.
   The medical device can effectively suppress the occurrence of whip.
(2) In the medical device, the requirement (A) may be satisfied, and x ≤ 8.0 may be satisfied. According to this configuration, a risk of vascular perforation can be effectively reduced.
(3) In the medical device, x ≤ 5.0 may be satisfied. According to this configuration, the risk of vascular perforation can be more effectively reduced.
(4) In the medical device, x ≤ 4.5 may be satisfied. According to this configuration, the risk of vascular perforation can be even more effectively reduced.
(5) In the medical device, the requirement (A) may be satisfied, and y ≥ 0.025 may be satisfied. According to this configuration, rotation performance is improved.
(6) In the medical device, y ≥ 0.030 may be satisfied. According to this configuration, rotation performance is improved.
(7) In the medical device, y ≥ 0.040 may be satisfied. According to this configuration, rotation performance is improved.
(8) In the medical device, the requirement (A) may be satisfied, and the requirement (B) may be satisfied. According to this configuration, a reduction in the risk of vascular perforation and an improvement in rotation performance are achieved.
(9) The medical device may further include a coil that covers the core shaft.
(10) In the medical device, the coil is an outer layer coil, and the medical device may further include an inner layer coil that covers the core shaft and is disposed inside the outer layer coil, and a distal tip that joins a distal end of the core shaft, a distal end of the outer layer coil, and a distal end of the inner layer coil.

The technology disclosed herein can be realized in various aspects, for example, in aspects such as a medical device and a method for manufacturing the same.

### BRIEF DESCRIPTION OF DRAWINGS

FIG. 1 is an explanatory view schematically illustrating a configuration of a guidewire in an embodiment.
FIG. 2 is an explanatory view illustrating a method for measuring a distal-end load of a guidewire.
FIG. 3 is an explanatory view illustrating a method for measuring a torque value of a guidewire.
FIG. 4 is a graph illustrating performance evaluation results.
FIG. 5 is an explanatory view illustrating a method for measuring a whip angle.

### EMBODIMENTS OF THE INVENTION

### A. Embodiment

### (Configuration of Guidewire 100)

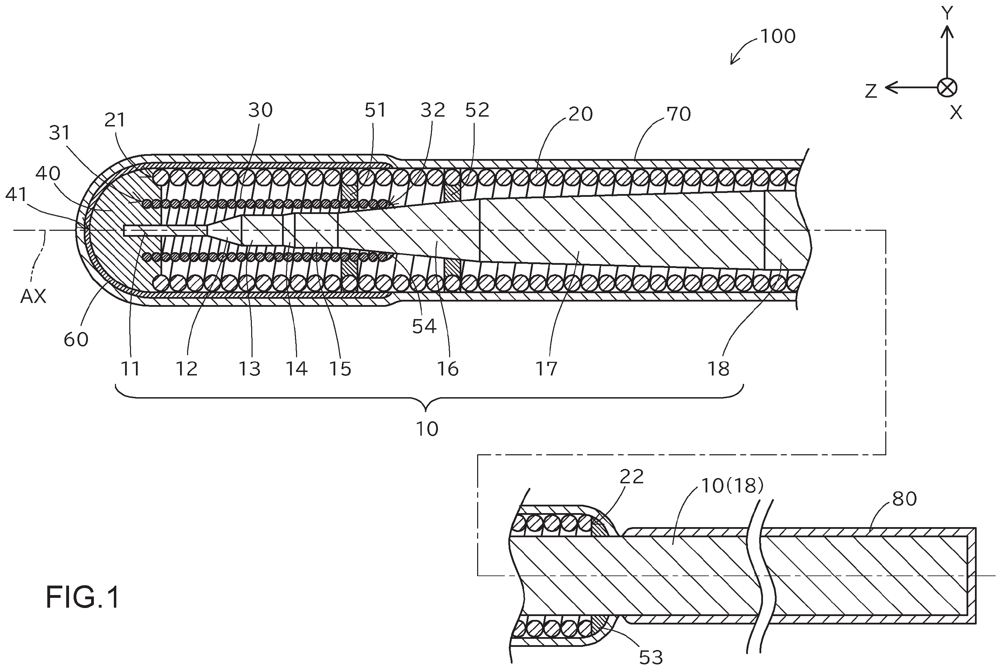

FIG. 1 is an explanatory diagram schematically illustrating a configuration of a guidewire 100 according to an embodiment. FIG. 1 shows XYZ axes orthogonal to one another for specifying directions, and shows a longitudinal section (YZ section) of the guidewire 100. Along a direction parallel to a central axis AX of the guidewire 100 (hereinafter referred to as an "axial direction"), a Z-axis positive direction side is a distal side (distal end side) to be inserted into a body, and a Z-axis negative direction side is a proximal side (proximal end side) to be operated by a physician. FIG. 1 shows a state in which the guidewire 100 has a linear shape substantially parallel to the Z-axis direction as a whole. The guidewire 100 has flexibility to an extent that allows bending. In this specification, regarding the guidewire 100 and each component thereof, an end on the distal side is referred to as a "distal end," the distal end and its vicinity are referred to as a "distal end portion," an end on the proximal side is referred to as a "proximal end," and the proximal end and its vicinity are referred to as a "proximal end portion."

The guidewire 100 is a medical device. The guidewire 100 is inserted into a blood vessel, for example, to guide another medical device (not shown) such as a catheter to a lesion portion in the blood vessel. The guidewire 100 includes a core shaft 10, an outer layer coil 20, an inner layer coil 30, a distal tip 40, a first intermediate joining portion 51, a second intermediate joining portion 52, a first proximal side joining portion 53, a second proximal side joining portion 54, a first coating 60, a second coating 70, and a third coating 80.

The core shaft 10 is an elongated member. A central axis of the core shaft 10 substantially coincides with the central axis AX of the guidewire 100. The core shaft 10 has a first portion 11, a second portion 12, a third portion 13, a fourth portion 14, a fifth portion 15, a sixth portion 16, a seventh portion 17, and an eighth portion 18. The first portion 11, the second portion 12, the third portion 13, the fourth portion 14, the fifth portion 15, the sixth portion 16, the seventh portion 17, and the eighth portion 18 are arranged in this order from the distal end toward the proximal side.

The first portion 11, the third portion 13, the fifth portion 15, and the eighth portion 18 of the core shaft 10 have a constant cross-sectional shape (XY section) at each position along the axial direction. An area of the cross section of the third portion 13 is larger than an area of the cross section of the first portion 11. An area of the cross section of the fifth portion 15 is larger than the area of the cross section of the third portion 13. An area of the cross section of the eighth portion 18 is larger than the area of the cross section of the fifth portion 15. The second portion 12, the fourth portion 14, the sixth portion 16, and the seventh portion 17 of the core shaft 10 smoothly connect cross-sectional shapes of other adjacent portions along the axial direction. The second portion 12, the fourth portion 14, the sixth portion 16, and the seventh portion 17 are tapered portions in which the area of the cross section gradually increases from the distal side toward the proximal side.

The core shaft 10 having such a shape can be manufactured, for example, by performing press working on a precursor having a constant cross section along the axial direction at a press rate corresponding to the shape of each portion of the core shaft 10. The press rate of the first portion 11 is 40%. The press rate of the third portion 13 is 6%.

Examples of a material for forming the core shaft 10 include metal materials, and more specifically, stainless steel (SUS302, SUS304, SUS316, etc.), nickel-titanium alloys, piano wire, nickel-chromium alloys, cobalt alloys, tungsten, and the like.

The outer layer coil 20 is a member formed in a hollow cylindrical shape by helically winding a wire. The outer layer coil 20 is, for example, a closely wound coil. The outer layer coil 20 is arranged so as to surround an outer periphery of the distal end portion of the core shaft 10. In the axial direction, a position of a distal end 21 of the outer layer coil 20 is substantially the same as the position of the distal end of the core shaft 10. The outer layer coil 20 is an example of a coil.

The inner layer coil 30 is a member formed in a hollow cylindrical shape by helically winding a wire. The inner layer coil 30 is, for example, a closely wound coil. The inner layer coil 30 is arranged so as to surround the outer periphery of the distal end portion of the core shaft 10 in a space inside the outer layer coil 20. In the axial direction, a position of a distal end 31 of the inner layer coil 30 is substantially the same as the position of the distal end of the core shaft 10, and a position of a proximal end 32 of the inner layer coil 30 is on the distal side of the position of the proximal end 22 of the outer layer coil 20.

Outer diameters and inner diameters of the outer layer coil 20 and the inner layer coil 30 may be constant along the axial direction or may change along the axial direction.

Examples of materials for forming the outer layer coil 20 and the inner layer coil 30 include metal materials, more specifically, radiolucent alloys such as stainless steel (SUS302, SUS304, SUS316, etc.), nickel-titanium alloys, piano wire, nickel-chromium alloys, or cobalt alloys, and radiopaque alloys such as gold, platinum, tungsten, or alloys containing these elements (e.g., platinum-nickel alloys).

The distal tip 40 joins the distal end of the core shaft 10 and the distal ends of the outer layer coil 20 and the inner layer coil 30. That is, the outer layer coil 20 and the inner layer coil 30 are connected to the distal tip 40. An outer peripheral surface of the distal side of the distal tip 40 is a smooth surface (for example, a substantially hemispherical surface). The first proximal side joining portion 53 joins the core shaft 10 and the proximal end portion of the outer layer coil 20. The second proximal side joining portion 54 joins the core shaft 10 and the proximal end portion of the inner layer coil 30. The first intermediate joining portion 51 joins the core shaft 10, an intermediate portion of the outer layer coil 20 (a portion excluding the distal end portion and the proximal end portion, the same applies hereinafter), and an intermediate portion of the inner layer coil 30. The second intermediate joining portion 52 is located on the proximal side of the first intermediate joining portion 51 and joins the core shaft 10 and the intermediate portion of the outer layer coil 20. Examples of materials for forming the distal tip 40 and the respective joining portions 51, 52, 53, 54 include metal solders such as silver solder, gold solder, zinc, Sn-Ag alloys, and Au-Sn alloys, and adhesives such as epoxy adhesives.

The first coating 60 covers the outer peripheral surface of the distal tip 40 and the outer peripheral surface of the outer layer coil 20 in a region from the distal end of the guidewire 100 to a predetermined position in the axial direction. Examples of materials for forming the first coating 60 include urethane resin.

The second coating 70 covers the outer peripheral surface of the distal tip 40 and the outer peripheral surface of the outer layer coil 20 in the region from the distal end of the guidewire 100 to the predetermined position in the axial direction. More specifically, in a region where the outer peripheral surface of the distal tip 40 and the outer peripheral surface of the outer layer coil 20 are covered with the first coating 60, the second coating 70 covers the outer peripheral surface of the distal tip 40 and the outer peripheral surface of the outer layer coil 20 via the first coating 60 by covering the first coating 60. In a region adjacent to the proximal side of the region where the outer peripheral surface of the outer layer coil 20 is not covered with the first coating 60, the second coating 70 directly covers the outer peripheral surface of the outer layer coil 20. Such a configuration can be realized, for example, by forming the first coating 60 in a predetermined region and then forming the second coating 70 in a region including the region. The second coating 70 is, for example, a hydrophilic coating. Examples of materials forming the second coating 70 include polyvinylpyrrolidone, polyacrylic acid, polyacrylamide, polyvinyl alcohol, maleic anhydride copolymer, hyaluronic acid, and the like.

The third coating 80 covers the outer peripheral surface of the core shaft 10 in a region on the proximal side of the proximal end 22 of the outer layer coil 20 in the guidewire 100. Examples of materials forming the third coating 80 include PTFE.

### (Method for Measuring Distal-End Load of Guidewire 100)

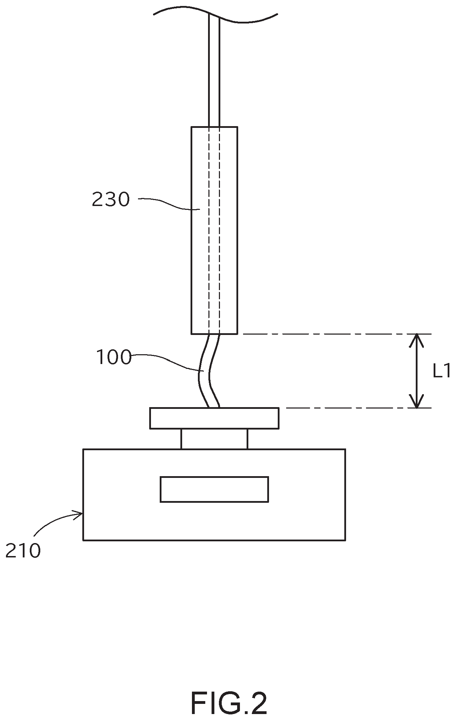

FIG. 2 is an explanatory view illustrating a method for measuring the distal-end load of the guidewire 100. First, an operator sets a tubular body 230 above an electronic balance 210. At this time, a shortest distance L1 from the electronic balance 210 to the tubular body 230 is set to 10.5 mm. The operator inserts the guidewire 100 into the tubular body 230 from above and makes the distal end portion of the guidewire 100 protrude from the lower end of the tubular body 230. In this state, the operator obtains a load measured by the electronic balance 210 when the distal end of the guidewire 100 is pressed against the electronic balance 210 as the distal-end load of the guidewire 100.

### (Method for Measuring Torque Value of Guidewire 100)

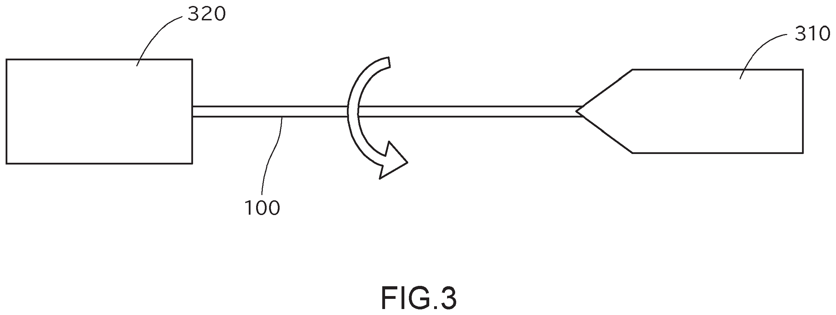

FIG. 3 is an explanatory view illustrating a method for measuring the torque value of the guidewire 100. First, the operator chucks the proximal end portion of the guidewire 100 to a rotating device 310 and chucks the distal end portion of the guidewire 100 to a torque sensor 320. In this state, the proximal side of the guidewire 100 is rotated twice by the rotating device 310. The operator obtains the load measured by the torque sensor 320 at this time as the torque value of the guidewire 100.

### (Characteristics of Guidewire 100)

The guidewire 100 of the embodiment has a relatively small distal-end load and a relatively large measured torque value. Specifically, the guidewire 100 is configured to satisfy at least one of the following requirement (A) and requirement (B). Requirement (A): y ≥ 0.0056x (where x is the distal-end load (mN), and y is the measured torque value (mN·m)). Requirement (B): x ≤ 4.5 and y ≥ 0.020.

The guidewire 100 may be configured to satisfy the requirement (A) and x ≤ 8.0. The guidewire 100 may be configured to satisfy the requirement (A) and x ≤ 5.0. The guidewire 100 may be configured to satisfy the requirement (A) and x ≤ 4.5.

The guidewire 100 may be configured to satisfy the requirement (A) and y ≥ 0.025. The guidewire 100 may be configured to satisfy the requirement (A) and y ≥ 0.030. The guidewire 100 may be configured to satisfy the requirement (A) and y ≥ 0.040.

Such characteristics of the guidewire 100 can be realized, for example, by adjusting the shape (outer diameter, length, etc.), material, and the like of the core shaft 10. Such characteristics of the guidewire 100 can also be realized by adjusting the shape (outer diameter, inner diameter, wire diameter, length, etc.), winding method, arrangement, material, and the like of the outer layer coil 20 and/or the inner layer coil 30.

### (Examples)

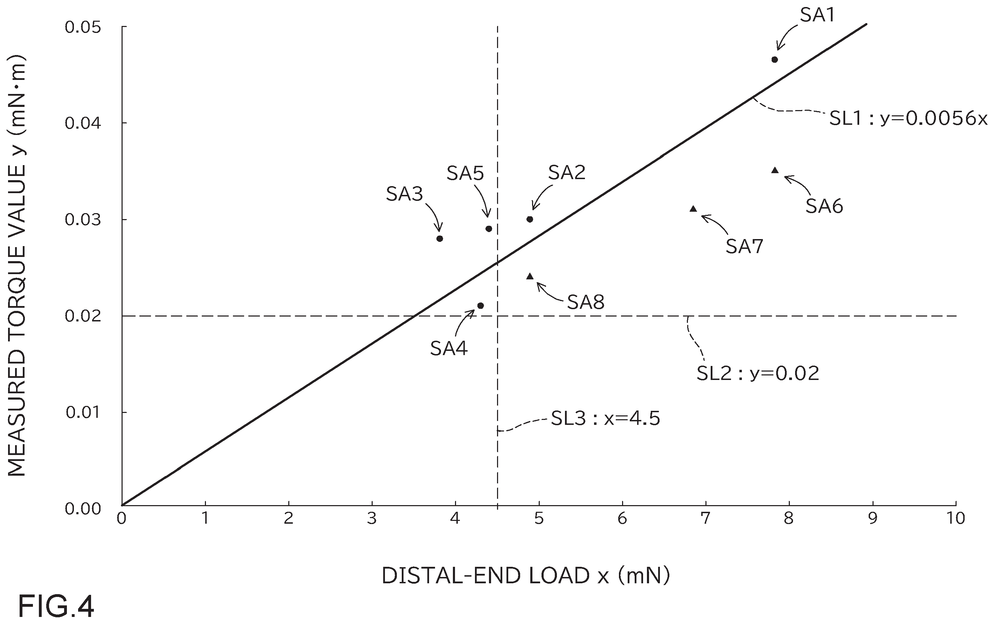

Eight samples (SA1 to SA8) of guidewires having configurations different from each other were prepared, and performance evaluation was performed for each sample. Table 1 is a table illustrating performance evaluation results, and FIG. 4 is a graph illustrating the performance evaluation results.

**[Table 1]**

| Sample No. | Distal-end load x (mN) | Measured torque value y (mN·m) | Whip angle (degree) |
|---|---|---|---|
| SA1 | 7.8 | 0.046 | 0 |
| SA2 | 4.9 | 0.030 | 0 |
| SA 3 | 3.8 | 0.028 | 0 |
| SA 4 | 4.3 | 0.021 | 0 |
| SA 5 | 4.4 | 0.029 | 0 |
| SA 6 | 7.8 | 0.035 | 65 |
| SA7 | 6.9 | 0.031 | 65 |
| SA8 | 4.9 | 0.024 | 135 |

In the performance evaluation, the distal-end load x and the torque value y of the guidewire 100 were measured according to the above-described measurement methods (see FIGS. 2 and 3).

In the performance evaluation, a whip angle of the guidewire 100 was measured. The whip angle is a rotation angle of the distal end portion of the guidewire 100 before and after the occurrence of a phenomenon called "whip" in which the distal end portion of the guidewire 100 does not rotate linearly even if the proximal end portion of the guidewire 100 is rotated, and the distal end portion rotates all at once when the proximal end portion is further rotated.

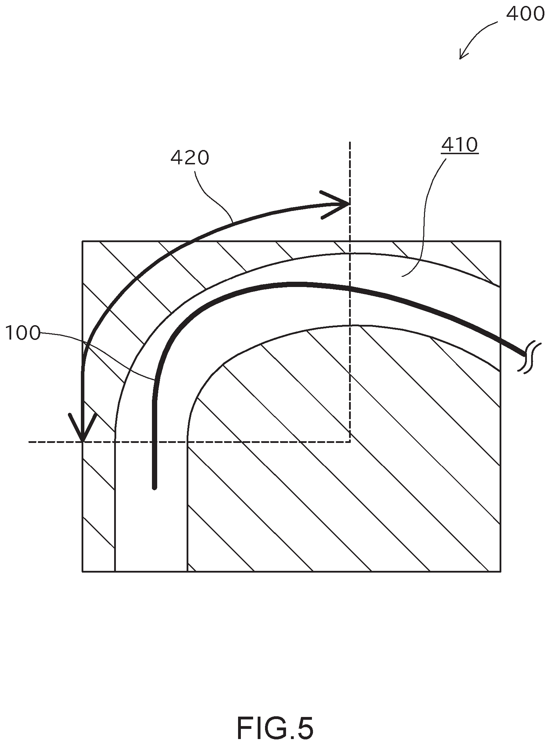

FIG. 5 is an explanatory view illustrating a method for measuring the whip angle. First, the operator performs shaping with a length of 7 mm and a height of 4 mm on the distal end portion of the guidewire 100. The length of shaping is the length along the axial direction of a region where shaping is performed in the guidewire 100, and the height of shaping is a distance along a direction orthogonal to the axial direction from the position of the distal end of the guidewire 100 before shaping to the position of the distal end of the guidewire 100 after shaping.

The operator inserts the shaped guidewire 100 into a hollow portion 410 of a jig 400. The hollow portion 410 is formed of silicone and has an inner diameter of 3.5 mm. A part of the hollow portion 410 (hereinafter referred to as a "bent portion 420") is bent. The operator makes a region of 5 mm at the distal end of the guidewire 100 protrude toward the distal side of the bent portion 420. In the guidewire 100, a portion on the proximal side of the portion inserted into the jig 400 is straightened. The operator takes an image with a camera from the distal side of the guidewire 100 and rotates the proximal end portion of the guidewire 100 twice. At this time, it is confirmed whether whip occurred, and if whip occurred, the rotation angle of the distal end portion of the guidewire 100 is measured by referring to images before and after the occurrence of whip.

As illustrated in Table 1, whip did not occur in samples SA1 to SA5. Whip occurred in samples SA6 to SA8. As illustrated in FIG. 4, samples SA1 to SA5 in which whip did not occur satisfy at least one of the requirement (A) and requirement (B). Samples SA1 to SA5 satisfy at least one of requirement (A') and requirement (B). Requirement (A') is y > 0.0056x. Samples SA1 to SA3 and SA5 satisfy the requirement (A) and the requirement (A'). Samples SA3 to SA5 satisfy the requirement (B). Samples SA3 and SA5 satisfy both of the requirements (A) and (B), and also satisfy the requirement (A'). Samples SA6 to SA8 in which whip occurred satisfy neither the requirement (A) nor the requirement (B).

Among the samples in which whip did not occur, samples SA1 to SA3 and SA5 satisfy the requirement (A) and x ≤ 8.0. Among the samples in which whip did not occur, samples SA2 to SA3 and SA5 satisfy the requirement (A) and x ≤ 5.0. Among the samples in which whip did not occur, samples SA3 and SA5 satisfy the requirement (A) and x ≤ 4.5.

Among the samples in which whip did not occur, samples SA1 to SA3 and SA5 satisfy the requirement (A) and y ≥ 0.025. Among the samples in which whip did not occur, samples SA1 to SA2 satisfy the requirement (A) and y ≥ 0.030. Among the samples in which whip did not occur, sample SA1 satisfies the requirement (A) and y ≥ 0.040.

### (Effect of Embodiment)

As described above, the guidewire 100 of the embodiment includes the core shaft 10 and satisfies at least one of the requirement (A) and the requirement (B), whereby the occurrence of whip can be effectively suppressed. The effect is obtained by the relationship between the distal-end load and the measured torque value, not by the cross-sectional shape of the core shaft 10. Even if the cross-sectional shape of the core shaft 10 is close to a round shape, which tends to improve rotation performance, whip may occur if the distal end is shaped. The effect is useful for suppressing whip in such a case. If the distal-end load is 8 mN or less, the risk of vascular perforation is low, and if the measured torque value is 0.025 mN·m or more, the rotation performance is excellent. Therefore, the guidewire 100 is suitable for a procedure of passing through a strut of a stent to select a branched blood vessel. The guidewire 100 having these features is also suitable as a workhorse wire.

### B. Modifications

The technology disclosed herein is not limited to the above-described embodiment, and can be modified into various modifications without departing from the gist thereof. For example, the following modifications are also possible.

The configuration of the guidewire 100 in the embodiment is merely an example and can be variously modified. For example, the guidewire 100 may not include the outer layer coil 20 and/or the inner layer coil 30, or the guidewire 100 may include other coils other than the outer layer coil 20 and the inner layer coil 30.

The guidewire 100 may not include the first coating 60, the second coating 70, and/or the third coating 80, or the guidewire 100 may include other coatings other than the first coating 60, the second coating 70, and the third coating 80.

The technology disclosed in the present specification is applicable not only to the guidewire 100 but also to medical devices in general.

## Claims

1. A medical device (100) comprising: a core shaft (10),
wherein the medical device (100) satisfies at least one of the following requirement (A) and requirement (B):
Requirement (A): y ≥ 0.0056x (where x is a distal-end load (mN) and y is a measured torque value (mN·m)); and
Requirement (B): x ≤ 4.5 and y ≥ 0.020.

2. The medical device (100) according to claim 1, wherein the medical device (100) satisfies the requirement (A) and satisfies x ≤ 8.0.

3. The medical device (100) according to claim 2, wherein the medical device (100) satisfies x ≤ 5.0.

4. The medical device (100) according to claim 2 or 3, wherein the medical device (100) satisfies x ≤ 4.5.

5. The medical device (100) according to any one of claims 1 to 4, wherein the medical device (100) satisfies the requirement (A) and satisfies y ≥ 0.025.

6. The medical device (100) according to claim 5, wherein the medical device (100) satisfies y ≥ 0.030.

7. The medical device (100) according to claim 5, wherein the medical device (100) satisfies y ≥ 0.040.

8. The medical device (100) according to any one of claims 1 to 7, wherein the medical device (100) satisfies the requirement (A) and satisfies the requirement (B).

9. The medical device (100) according to any one of claims 1 to 8, further comprising a coil (20) covering the core shaft (10).

10. The medical device (100) according to claim 9,
wherein the coil (20) is an outer layer coil (20), and
the medical device (100) further comprises:
an inner layer coil (30) that covers the core shaft (10) and is disposed inside the outer layer coil (20); and
a distal tip (40) that joins a distal end of the core shaft (10), a distal end of the outer layer coil (20), and a distal end of the inner layer coil (30).
